# EUROPÄISCHE PATENTANMELDUNG

(11) **EP 4 844 237 A2**
(43) Veröffentlichungstag der Anmeldung: **25.11.2026**
(21) Anmeldenummer: 26210682.6
(22) Anmeldetag: 25.02.2025
(51) Int. Cl.: F16B 21/04

(54) **BEFESTIGUNG EINER HEIZ- UND/ODER TEMPERATURMESSVORRICHTUNG IN UND/ODER AN EINEM MEDIZINGERÄT**

(30) Priorität: 26.02.2024 DE 102024105354
(62) Dokumentnummer(n) der früheren Anmeldung(en) nach Art. 76 EPÜ: 25159998.1 / 4 606 411
(71) Anmelder: Löwenstein Medical Technology S.A., 2557 Luxembourg (LU)
(72) Erfinder: Göbel, Christof, Hamburg (DE); Beer, Maike, Hamburg (DE); Hahn, Henry, Hamburg (DE); Hein, Stefan, Hamburg (DE); Sepke, Heiko, Lütjensee (DE)
(74) Vertreter: Löwenstein Medical IP

(57) **Zusammenfassung**

Eine Befestigungsanordnung (1) für ein Medizingerät umfasst: ein an einem Befestigungsabschnitt des Medizingeräts befestigbares Aufnahmeelement (8); ein bis in eine Dichtstellung in das Aufnahmeelement einführbares Einführelement (6) mit einer Heiz- und/oder Temperaturmessvorrichtung (2, 4) zum Erwärmen und/oder Messen einer Temperatur einer Flüssigkeit und/oder eines Gases in einer Flüssigkeitskammer (30) des Medizingeräts, wobei das Einführelement in der Dichtstellung fluiddicht mit dem Aufnahmeelement verbunden ist; ein Verbindungselement (10), das eine relativ zum Aufnahmeelement und/oder zum Einführelement zwischen einer Verriegelungsstellung und einer Entriegelungsstellung bewegliche Komponente (12) umfasst, wobei die bewegliche Komponente ausgebildet ist, um das bis in die Dichtstellung in das Aufnahmeelement eingeführte Einführelement in der Verriegelungsstellung zu verriegeln und in der Entriegelungsstellung freizugeben, wobei die bewegliche Komponente relativ zum Aufnahmeelement und/oder zum Einführelement zwischen der Verriegelungsstellung und der Entriegelungsstellung um einen Drehwinkel von höchstens 360°, höchstens 180° oder höchstens 90° um eine Drehachse (24) drehbar gelagert ist.

## Beschreibung

### Technisches Gebiet

Die vorliegende Erfindung betrifft eine Befestigungsanordnung zum Befestigen einer Heiz- und/oder Temperaturmessvorrichtung in und/oder an einem Medizingerät. Des Weiteren betrifft die Erfindung ein mit einer solchen Befestigungsanordnung ausgestattetes Medizingerät.

### Stand der Technik

Ein Medizingerät in Form eines Atemtherapiegeräts kann zur Behandlung schlafbezogener Atmungsstörungen oder anderweitiger Probleme mit der Atempumpe eingesetzt werden. Hierbei ist ein Patient über ein geeignetes Patienteninterface, beispielsweise in Form einer Gesichts- oder Nasenmaske, und einen Patientenschlauch mit dem Atemtherapiegerät verbunden. Um das Risiko von pulmonalen Infektionen oder Schädigungen des Lungengewebes zu verringern, kann es erforderlich sein, das dem Pateinten zugeführte Atemgas in geeigneter Weise zu konditionieren. Unter Atemgaskonditionierung wird in der Regel die Vorbereitung des Atemgases durch dessen Erwärmung und/oder Reinigung verstanden, was insbesondere bei empfindlichen Patienten mit vorgeschädigten Lungen wichtig ist. Dazu kann ein aktiver Atemgasbefeuchter - beispielsweise ein Vernebler, Verdunster oder Sprudler - oder ein passiver Atemgasbefeuchter, auch *heat and moisture exchanger* oder kurz HME genannt, eingesetzt werden.

Ein Atemgasbefeuchter kann eine Flüssigkeitskammer mit erwärmtem und/oder gereinigtem Wasser umfassen, über das Atemgas geleitet und somit erwärmt bzw. befeuchtet werden kann. Die Flüssigkeitskammer kann eine (interne) Heizvorrichtung zum Erwärmen des Wassers, beispielsweise in Form eines Heizstabs, und/oder eine Temperaturmessvorrichtung zum Messen der Temperatur der Flüssigkeit, beispielsweise in Form eines Tauchrohrs, aufweisen. Möglich sind auch externe Heizvorrichtungen.

Eine solche Heiz- und/oder Temperaturmessvorrichtung kann beispielsweise über eine Schraubverbindung an der Flüssigkeitskammer befestigt werden.

Der Atemgasbefeuchter sollte regelmäßig gereinigt werden, weil im erwärmten Wasser in der Flüssigkeitskammer Bakterien und Pilze wachsen können. Für eine regelmäßige gründliche Reinigung der Flüssigkeitskammer und/oder der Heiz- und/oder Temperaturmessvorrichtung ist es zweckmäßig, wenn die entsprechenden Komponenten vom Nutzer demontiert werden können, sodass die Komponenten separat gereinigt werden können, insbesondere auch die jeweiligen Gewindeabschnitte.

Um die Heiz- und/oder Temperaturmessvorrichtung einzuschrauben, sodass die resultierende Schraubverbindung wasserdicht ist, oder auszuschrauben, sind in der Regel beide Hände nötig. Zudem haben manche Nutzer Schwierigkeiten, das richtige Drehmoment beim Zusammenschrauben abzuschätzen. Ist das Drehmoment zu gering, kann es infolge einer undichten Schraubverbindung dazu kommen, dass Wasser aus der Flüssigkeitskammer austritt. Ist das Drehmoment zu groß, kann es zu Beschädigungen der Komponenten, insbesondere von Kunststoffteilen oder der Gewinde, kommen. Viele Beatmungspatienten sind zudem körperlich geschwächt und/oder leiden an zusätzlichen Erkrankungen wie Gicht, Arthrose oder Rheuma. Patienten mit derart eingeschränkter Motorik können sich beim Montieren bzw. Demontieren einer solchen schraubbaren Heiz- und/oder Temperaturmessvorrichtung besonders schwertun.

### Offenbarung der Erfindung

Eine Aufgabe der Erfindung kann darin gesehen werden, eine Befestigungsanordnung bereitzustellen, die eine komfortablere Montage bzw. Demontage der Heiz- und/oder Temperaturmessvorrichtung im Vergleich zu schraubbaren Varianten ermöglicht. Eine weitere Aufgabe der Erfindung kann darin gesehen werden, ein entsprechendes Medizingerät bereitzustellen.

Diese Aufgaben werden durch die Gegenstände der unabhängigen Ansprüche gelöst. Vorteilhafte Ausführungsformen sind in den abhängigen Ansprüchen, der nachfolgenden Beschreibung und den beigefügten Figuren dargelegt.

Ein erster Aspekt betrifft eine Befestigungsanordnung für ein Medizingerät, insbesondere einen Atemgasbefeuchter. Die Befestigungsanordnung umfasst: ein an einem Befestigungsabschnitt des Medizingeräts befestigbares Aufnahmeelement; ein in das Aufnahmeelement einführbares Einführelement, das eine Heiz- und/oder Temperaturmessvorrichtung zum Erwärmen und/oder Messen einer Temperatur einer Flüssigkeit und/oder eines Gases in einer Flüssigkeitskammer des Medizingeräts umfasst; ein Verbindungselement zum Verbinden des Einführelements mit dem Aufnahmeelement, wobei das Verbindungselement eine relativ zum Aufnahmeelement und/oder zum Einführelement zwischen einer Verriegelungsstellung und einer Entriegelungsstellung bewegliche Komponente umfasst. Die bewegliche Komponente ist ausgebildet, um das in das Aufnahmeelement eingeführte Einführelement in der Verriegelungsstellung zu verriegeln, sodass ein Entnehmen (oder ein Herausfallen) des Einführelements aus dem Aufnahmeelement nicht möglich ist, und in der Entriegelungsstellung freizugeben, sodass das Entnehmen möglich ist.

Eine solche Befestigungsanordnung ermöglicht auch Patienten mit eingeschränkter Motorik eine einfache und mühelose Montage bzw. Demontage der Heiz- und/oder Temperaturmessvorrichtung, beispielsweise zu Reinigungszwecken, ohne dass Beschädigungen oder Leckagen auftreten, wie dies häufig bei schraubbaren Varianten der Fall ist.

Die bewegliche Komponente kann ausgebildet sein, um in der Verriegelungsstellung eine form- und/oder kraftschlüssige Verbindung zwischen dem Aufnahmeelement und dem in das Aufnahmeelement eingeführten Einführelement herzustellen und die form- und/oder kraftschlüssige Verbindung in der Entriegelungsstellung wieder zu lösen. Die form- und/oder kraftschlüssige Verbindung kann so ausgebildet sein, dass ein manuelles Entnehmen des Einführelements aus dem Aufnahmeelement (mit normalem Kraftaufwand) nicht möglich ist.

Die bewegliche Komponente kann am Aufnahmeelement und/oder am Einführelement und/oder an einem sonstigen Abschnitt der Befestigungsanordnung und/oder des Medizingeräts zwischen der Verriegelungs- und der Entriegelungsstellung beweglich gelagert sein.

Die bewegliche Komponente kann relativ zum Aufnahmeelement und/oder zum Einführelement zwischen der Verriegelungs- und der Entriegelungsstellung dreh- und/oder verschiebbar sein und/oder beim Bewegen zwischen der Verriegelungs- und der Entriegelungsstellung einer geraden und/oder gekrümmten Bahn folgt.

Beispielsweise kann die bewegliche Komponente fest mit dem Einführelement verbunden sein und zusammen mit dem Einführelement relativ zum Aufnahmeelement zwischen der Verriegelungs- und der Entriegelungsstellung beweglich, beispielsweise dreh- und/oder verschiebbar, sein. Alternativ kann die bewegliche Komponente fest mit dem Aufnahmeelement verbunden sein und zusammen mit dem Aufnahmeelement relativ zum Einführelement zwischen der Verriegelungs- und der Entriegelungsstellung beweglich, beispielsweise dreh- und/oder verschiebbar, sein.

Die bewegliche Komponente kann ausgebildet sein, um das in das Aufnahmeelement eingeführte Einführelement zumindest abschnittsweise zu umrahmen.

Das Aufnahmeelement kann beispielsweise als Teil eines Außengehäuses der Flüssigkeitskammer ausgebildet sein und/oder an einem Abschnitt (eines Außengehäuses) der Flüssigkeitskammer als dem Befestigungsabschnitt befestigbar sein.

Das Erwärmen bzw. Messen der Temperatur des Gases mittels der Heiz- und/oder Temperaturmessvorrichtung kann beispielsweise bei einem niedrigen Flüssigkeitsstand in der Flüssigkeitskammer relevant sein. Denkbar ist auch, dass zumindest ein zum Erwärmen bzw. Messen verwendeter Teil der Heiz- und/oder Temperaturmessvorrichtung zusätzlich in einen anderen Abschnitt des jeweiligen Medizingeräts hineinragt, wo dieser Teil nicht (oder zumindest nicht vollständig) mit der Flüssigkeit, sondern mit einem Gas (beispielsweise Umgebungsluft) Kontakt hat, sodass in diesem Fall zusätzlich oder alternativ dieses Gas erwärmt bzw. die Temperatur dieses Gases gemessen wird.

Ein zweiter Aspekt betrifft ein Medizingerät, insbesondere einen Atemgasbefeuchter. Das Medizingerät umfasst eine Flüssigkeitskammer, einen Befestigungsabschnitt und eine Befestigungsanordnung, wie sie vor- und nachstehend beschrieben wird, deren Aufnahmeelement am Befestigungsabschnitt des Medizingeräts befestigt ist.

Das am Befestigungsabschnitt befestigte Aufnahmeelement kann gegenüber einer Öffnung in einem Gehäuse der Flüssigkeitskammer derart ausgerichtet sein, dass die Heiz- und/oder Temperaturmessvorrichtung durch die Öffnung hindurch ins Innere der Flüssigkeitskammer ragt, wenn das Einführelement vollständig in das Aufnahmeelement eingeführt ist.

Zusätzlich kann das Medizingerät eine Befeuchtungsvorrichtung umfassen, die ausgebildet ist, um Atemgas unter Verwendung von Flüssigkeit aus der Flüssigkeitskammer zu befeuchten und das befeuchtete Atemgas an einem Anschluss für einen Beatmungsschlauch bereitzustellen.

Die Flüssigkeitskammer kann beispielsweise ein Wasserbehälter sein und/oder ein Fassungsvermögen zwischen 100 ml und 1000 ml, zwischen 600 ml und 800 ml oder zwischen 300 ml und 400 ml haben.

Bei dem Medizingerät kann es sich beispielsweise um einen Atemgasbefeuchter, ein Beatmungsgerät, insbesondere in Form eines CPAP- und/oder High-Flow-Atemtherapiegeräts, oder ein Gerät zur Sekretentfernung handeln.

Das Medizingerät kann ferner mindestens eine der folgenden Komponenten umfassen: eine Messsonde zum Messen eines Flusses und/oder einer Temperatur eines Atemgases; ein Schlauchsystem zum Leiten eines Atemgases hin zu und/oder weg von einem Patienten; eine Schlauchheizung zum Beheizen eines oder mehrerer Schläuche des Schlauchsystems.

Im Folgenden werden verschiedene Ausführungsformen beschrieben. Diese Ausführungsformen sind nicht als Beschränkung des Umfangs der Offenbarung zu verstehen.

Gemäß einer Ausführungsform kann die bewegliche Komponente zwischen der Verriegelungs- und der Entriegelungsstellung verschiebbar und/oder um einen Drehwinkel von höchstens 360°, höchstens 180°, höchstens 120° oder - bevorzugt - höchstens 90° um eine Drehachse (beispielsweise um eine Längsachse des Einführelements) drehbar gelagert sein. Anders ausgedrückt kann die Bewegung der beweglichen Komponente zwischen der Verriegelungs- und der Entriegelungsstellung eine reine Translation, eine reine Rotation oder eine Kombination aus einer Translation und einer Rotation sein. Dabei kann die Rotation auf einen bestimmten maximalen Drehwinkel, beispielsweise von höchstens 360°, höchstens 180°, höchstens 120° oder - bevorzugt - höchstens 90°, begrenzt sein. Dies kann die Bedienung der beweglichen Komponente vereinfachen, beispielsweise im Vergleich zu einer Ausführungsform mit einer Schraubverbindung, die in der Regel um mehr als eine volle Umdrehung angezogen wird.

Es ist möglich, dass das (beispielsweise längliche) Einführelement beim Einführen ausschließlich in einer Richtung parallel zu seiner Längsachse bewegt wird. Anders ausgedrückt kann das Einführen des Einführelements (im Gegensatz zu einem Einschrauben) ohne Verändern seiner Winkelstellung oder seines Drehwinkels in Bezug auf seine Längsachse als einer Drehachse erfolgen.

Gemäß einer Ausführungsform kann das Einführelement bis in eine Dichtstellung in das Aufnahmeelement einführbar sein und in der Dichtstellung fluiddicht mit dem Aufnahmeelement verbunden sein, beispielsweise derart, dass ein Spalt zwischen dem Einführelement und dem Aufnahmeelement so abgedichtet wird, dass keine Flüssigkeit und/oder kein Gas aus der Flüssigkeitskammer über den Spalt nach außen entweichen kann. In diesem Fall kann die bewegliche Komponente ausgebildet sein, um das in der Dichtstellung befindliche Einführelement in der Verriegelungsstellung zu verriegeln und/oder in der Entriegelungsstellung zu entriegeln. Beispielsweise kann die Befestigungsanordnung so ausgebildet sein, dass das Einführelement in der Dichtstellung mit einer bestimmten, zum Abdichten erforderlichen Kraft, die beispielsweise in Richtung einer Längsachse des Einführelements wirken kann, gegen das Aufnahmeelement gedrückt wird.

Gemäß einer Ausführungsform kann die Befestigungsanordnung so ausgebildet sein, dass das Einführen des Einführelements bis in die Dichtstellung ohne Drehen des Einführelements und/oder der beweglichen Komponente relativ zum Aufnahmeelement um mehr als 360°, mehr als 180°, mehr als 120° oder - bevorzugt - mehr als 90° um eine Drehachse möglich ist. Es ist auch möglich, dass das Einführen des Einführelements bis in die Dichtstellung durch bloßes Einschieben erfolgt.

Alternativ kann die Befestigungsanordnung so ausgebildet sein, dass sich das Aufnahmeelement beim Drehen des Einführelements um die Drehachse zumindest teilweise mit dem Einführelement mitdreht.

Im Vergleich zu einer Schraubverbindung, deren Dichtheit maßgeblich vom Drehmoment, mit der die Schraubverbindung festgezogen wird, abhängt, hat diese Ausführungsform den Vorteil, dass zum Herstellen der fluiddichten Verbindung weder eine größere Drehbewegung noch ein nennenswertes Drehmoment erforderlich ist. Dies kann die Montage stark vereinfachen und/oder die Befestigungsanordnung robuster gegen Montagefehler machen.

Gemäß einer Ausführungsform kann die Befestigungsanordnung ferner ein Dichtelement umfassen und so ausgebildet sein, dass das Dichtelement beim Einführen des Einführelements bis in die Dichtstellung einerseits durch das Einführelement und/oder das Verbindungselement und/oder die bewegliche Komponente und andererseits durch das Aufnahmeelement zusammengedrückt wird, um das Einführelement fluiddicht mit dem Aufnahmeelement zu verbinden.

Anders ausgedrückt kann das Dichtelement ausgebildet sein, um im zusammengedrückten Zustand einen Spalt zwischen dem Einführelement und dem Aufnahmeelement und/oder zwischen dem Verbindungselement und dem Aufnahmeelement und/oder zwischen der beweglichen Komponente und dem Aufnahmeelement fluiddicht zu verschließen, sodass keine Flüssigkeit (oder kein Gas) oder keine nennenswerte Menge an Flüssigkeit (oder an Gas) aus der Befestigungsanordnung austreten kann, wenn sich das Einführelement in der Dichtstellung befindet.

Gemäß einer Ausführungsform kann die bewegliche Komponente ausgebildet sein, um in der Verriegelungsstellung das Einführelement und/oder das Aufnahmeelement mit einer in Richtung der Dichtstellung wirkenden Kraft zu beaufschlagen. Dadurch kann die Dichtwirkung weiter verbessert werden. Beispielsweise kann die Kraft geeignet sein, das Dichtelement mit einem definierten Druck zusammenzudrücken, sodass die fluiddichte Verbindung auch bei größeren, beispielsweise fertigungs-, montage- oder temperaturbedingten Maß- und/oder Formabweichungen gewährleistet werden kann.

Gemäß einer Ausführungsform kann das bis in die Dichtstellung eingeführte Einführelement an mindestens einem Anschlag anliegen. Der Anschlag kann ausgebildet sein, um ein weiteres Einführen des Einführelements über die Dichtstellung hinaus zu verhindern. Auf diese Weise können Leckagen und/oder Beschädigungen durch zu tiefes Einführen des Einführelements vermieden werden.

Gemäß einer Ausführungsform kann die bewegliche Komponente, beispielsweise am Einführelement und/oder am Aufnahmeelement, derart federnd ausgebildet und/oder federnd gelagert sein, dass sie in der Entriegelungsstellung mit einer in Richtung der Verriegelungsstellung wirkenden Rückstellkraft beaufschlagt wird. Dies hat die Wirkung, dass sich die bewegliche Komponente von allein in die Verriegelungsstellung zurückbewegt, wenn sie nicht anderweitig daran gehindert wird. Dies kann die Montage bzw. Demontage weiter vereinfachen. Beispielsweise kann die bewegliche Komponente mittels eines separaten Federelements federnd gelagert sein. Zusätzlich oder alternativ kann die bewegliche Komponente selbst in geeigneter Weise elastisch verformbar ausgebildet sein und so die federnde Lagerung ermöglichen.

Gemäß einer Ausführungsform kann die Heiz- und/oder Temperaturmessvorrichtung mindestens einen der folgenden Bestandteile umfassen: ein (beispielsweise elektrisches) Heizelement zum Erwärmen der Flüssigkeit und/oder des Gases; einen Temperaturfühler zum Messen der Temperatur der Flüssigkeit und/oder des Gases. Ferner kann die Heiz- und/oder Temperaturmessvorrichtung mindestens einen der folgenden Bestandteile umfassen: ein elektrisches Kontaktelement zum elektrisch leitfähigen Kontaktieren des (elektrischen) Heizelements und/oder des Temperaturfühlers; ein Dichtelement zum Abdichten eines Spalts zwischen dem Aufnahmeelement und dem in das Aufnahmeelement eingeführten Einführelement (beispielsweise ein Dichtelement, wie es vorstehend beschrieben wird); ein Blockierelement, das ausgebildet ist, um eine maximale Einführtiefe des Einführelements beim Einführen in das Aufnahmeelement festzulegen.

Das Blockierelement kann als Teil des Einführelements den Nutzer daran hindern, das Einführelement zu weit in das Aufnahmeelement zu schieben. Das Blockierelement kann beispielsweise als ein erhöhter Abschnitt am Einführelement ausgebildet sein, der das Einführelement abschnittsweise oder komplett umschließen kann. Als Gegenpart zum Blockierelement kann die Befestigungsanordnung im Aufnahmeelement zusätzlich mindestens einen Vorsprung zum Blockieren des Blockierelements beim Einführen des Einführelements aufweisen, sodass ein weiteres Einführen nicht mehr möglich ist. Der Vorsprung kann beispielsweise in Form eines erhöhten Rings ausgebildet sein und/oder mindestens eine Ausbuchtung umfassen. Bei dem Blockierelement kann es sich beispielsweise um den vorstehend beschriebenen Anschlag handeln.

Das Heizelement kann ausgebildet sein, um elektrischen Strom kontrolliert in Wärme umzuwandeln und die Wärme auf die Flüssigkeit und/oder das Gas in der Flüssigkeitskammer zu übertragen, beispielsweise um die Flüssigkeit und/oder das Gas auf eine Temperatur zwischen 20° und 80° zu bringen. Zusätzlich kann das Heizelement mit einem Temperatursensor zum Messen einer Ist-Temperatur in der Flüssigkeitskammer gekoppelt sein und/oder über eine Sicherheitsschaltung automatisch deaktiviert werden, wenn erkannt wird, dass das Heizelement zu heiß wird, beispielsweise weil keine oder zu wenig Flüssigkeit oder kein oder zu wenig Gas in der Flüssigkeitskammer vorhanden ist.

Das Dichtelement kann beispielsweise aus mindestens einem der folgenden Dichtmaterialien gefertigt sein: Ethylen-Propylen-Dien-Kautschuk, Chlorbutadien-Kautschuk, Naturkautschuk, Ethylen-Vinylacetat-Kautschuk, Styrol-Butadien-Kautschuk, Acrylnitril-Butadien-Kautschuk, Polyurethan-Kautschuk, Polytetrafluorethylen, Silikon, Silikonschaum, einem Elastomer mit Faseranteil. Das Dichtelement kann beispielsweise ein Dichtring sein. In diesem Fall kann die Heiz- und/oder Temperaturmessvorrichtung eine ringförmige Verjüngung zum Aufnehmen des Dichtrings umfassen. Die Verjüngung kann beispielsweise als Materialaussparung im Einführelement ausgebildet sein. Das Dichtelement kann auch eine Dichtmasse sein.

Zumindest ein Teil der Heiz- und/oder Temperaturmessvorrichtung, beispielsweise zumindest ein Abschnitt des elektrischen Kontaktelements, kann außerhalb der Flüssigkeitskammer liegen, wenn das Einführelement (vollständig) in das Aufnahmeelement eingeführt ist.

Gemäß einer Ausführungsform können mindestens zwei der Bestandteile oder alle Bestandteile der Heiz- und/oder Temperaturmessvorrichtung in Richtung deren Längsachse betrachtet hintereinander angeordnet sein. Dabei kann die Heiz- und/oder Temperaturmessvorrichtung länglich, beispielsweise stabförmig, ausgebildet sein. Beispielsweise kann das Dichtelement zwischen dem Heizelement und dem Blockierelement und/oder zwischen dem Temperaturfühler und dem Blockierelement angeordnet sein und/oder kann das Blockierelement zwischen dem Dichtelement und dem elektrischen Kontaktelement angeordnet sein.

Gemäß einer Ausführungsform kann das Aufnahmeelement mindestens einen Vorsprung aufweisen, der ausgebildet ist, um beim Einführen des Einführelements in das Aufnahmeelement gegen das Blockierelement zu stoßen, sodass ein weiteres Einführen des Einführelements verhindert wird.

Gemäß einer Ausführungsform kann das Aufnahmeelement als ein Kanal, insbesondere als ein zylinderförmiger Kanal, mit einer Einführseite, einer Begrenzungsseite, einer Ummantelung und einer Innenwandung ausgebildet sein. In diesem Fall kann der Vorsprung an der Begrenzungsseite und/oder an der Innenwandung angeordnet sein. Unter "Ummantelung" kann beispielsweise ein Element verstanden werden, das einen Längsabschnitt des Kanals in Richtung dessen Umfangs zumindest abschnittsweise ummantelt. Die Ummantelung kann den Längsabschnitt in Richtung dessen Umfangs auch vollständig ummanteln und/oder beispielsweise ring-, schalen- und/oder kragenförmig ausgebildet sein. Unter "Innenwandung" kann beispielsweise zumindest ein Abschnitt einer inneren Mantelfläche des Kanals verstanden werden. Der Kanal kann beispielsweise den gleichen oder einen ähnlichen Querschnitt wie das Einführelement haben.

Beispielsweise können die beiden Querschnitte komplementär zueinander ausgeformt sein und/oder kann ein Innendurchmesser (einer inneren Mantelfläche) des Kanals geringfügig größer als ein Außendurchmesser (einer äußeren Mantelfläche) des Einführelements sein, sodass das Einführelement spielfrei oder mit einem definierten Spiel in das Aufnahmeelement einführbar ist. Das Einführelement kann von der Einführseite aus in das Aufnahmeelement einführbar sein. Die Begrenzungsseite kann - beispielsweise in Richtung einer Mittel- oder Längsachse des Kanals betrachtet - der Einführseite gegenüberliegen.

Gemäß einer Ausführungsform kann das Verbindungselement, insbesondere die bewegliche Komponente, mindestens ein Einrastelement - beispielsweise in Form einer Nase oder eines Hakens - zum Verrasten des Einführelements mit dem Aufnahmeelement umfassen. Das Einrastelement kann beispielsweise ausgebildet sein, um das Einführelement mit dem Aufnahmeelement beim Bewegen der beweglichen Komponente in die Verriegelungsstellung zu verrasten und/oder die Verrastung des Einführelements mit dem Aufnahmeelement beim Bewegen der beweglichen Komponente in die Entriegelungsstellung zu lösen. Das Einrastelement kann zwischen der Einführseite und der Begrenzungsseite und/oder zwischen der Einführseite und dem Vorsprung angeordnet sein. Zudem kann das Einrastelement im Zustand des nicht eingeführten Einführelements einen ausgehend von der Innenwandung in den Kanal ragenden Austritt bilden und so ausgebildet sein, dass es beim Einführen des Einführelements in das Aufnahmeelement durch das Einführelement und/oder durch manuelles (beispielsweise mit einem Finger) Betätigen eines Druckelements zum Ausüben eines Drucks auf den Austritt in die Ummantelung gedrückt wird, sodass der Austritt nicht mehr in den Kanal ragt. Beispielsweise kann ein Zustand, in dem das Einrastelement in die Ummantelung aufgenommen ist, der Entriegelungsstellung entsprechen. Hingegen kann das Einrastelement in der Verriegelungsstellung formschlüssig in das Einführelement eingreifen und/oder mit dem Einführelement verhakt sein.

Es ist möglich, dass das Druckelement zusammen mit dem Einrastelement das Verbindungselement, insbesondere die bewegliche Komponente, bildet. Beispielsweise kann das mit dem Einrastelement in Verbindung stehende Druckelement während des Einführens des Einführelements von einem Nutzer betätigbar sein.

Gemäß einer Ausführungsform kann das Einführelement ferner einen Halteabschnitt zum Halten des Einführelements mit einer Hand und/oder kann das Verbindungselement, beispielsweise die bewegliche Komponente, ferner einen Halteabschnitt zum Halten des Verbindungselements bzw. der beweglichen Komponente mit einer Hand umfassen. Der Halteabschnitt kann so ausgebildet sein, dass er zumindest teilweise außerhalb des Aufnahmeelements liegt, wenn das Einführelement - beispielsweise bis in die Dichtstellung - in das Aufnahmeelement eingeführt ist. Beispielsweise kann der Halteabschnitt entlang der Längsachse der Heiz- und/oder Temperaturmessvorrichtung und/oder des Einführelements neben dem elektrischen Kontaktelement und/oder zwischen dem elektrischen Kontaktelement und dem Blockierelement angeordnet sein. An diesem Halteabschnitt soll ein Nutzer das Einführelement und/oder das Verbindungselement (beispielsweise die bewegliche Komponente) gefahrlos greifen können, ohne die Heiz- und/oder Temperaturmessvorrichtung zu berühren. Zusätzlich kann die bewegliche Komponente zumindest teilweise den Halteabschnitt des Einführelements umgeben und so ausgebildet sein, dass ein Nutzer auch die bewegliche Komponente gefahrlos greifen kann.

Gemäß einer Ausführungsform kann der Halteabschnitt umfassen: einen ringförmigen Grundkörper zum Aufschieben auf das Einführelement (beispielsweise auf die Heiz- und/oder Temperaturmessvorrichtung); einen in radialer Richtung vom Grundkörper abstehenden Haltearm zum Bewegen (beispielsweise Drehen und/oder Verschieben) des Grundkörpers relativ zum Einführelement (und/oder zum Aufnahmeelement).

Der Haltearm kann beispielsweise an seinem freien Ende ein flächiges Griffelement zum Greifen des Haltearms mit einem Daumen und einem Zeigefinger aufweisen. Anders ausgedrückt kann der Haltearm beispielsweise fahnenartig ausgebildet sein. Es ist möglich, dass das Verbindungselement und/oder die bewegliche Komponente zumindest teilweise durch den Grundkörper gebildet ist bzw. sind. Auf und/oder an dem Halteabschnitt, vorzugsweise auf und/oder an dem Haltearm und/oder dem flächigen Griffelement, kann beispielsweise ein entsprechender Warn- und/oder Bedienhinweis angebracht sein. Ein solcher Halteabschnitt bildet einen besonders langen Hebelarm zum Ver- bzw. Entriegeln des Einführelements und/oder zum Verpressen des Dichtelements. Des Weiteren kann ein solcher Halteabschnitt als eine besonders gut ablesbare, eindeutige Positionsanzeige für die Ver- bzw. Entriegelungsstellung fungieren. Dies kann den Bedienkomfort weiter verbessern.

Zusätzlich kann der Halteabschnitt zwischen einer Blockierstellung und einer Freigabestellung relativ zum Einführelement und/oder zum Aufnahmeelement bewegbar sein und ausgebildet sein, um eine mechanische Kopplung des Medizingeräts, beispielsweise eines Beatmungsgeräts, mit einem weiteren Gerät, beispielsweise einem Atemluftbefeuchter, in der Blockierstellung zu blockieren und in der Freigabestellung zu ermöglichen. Es ist möglich, dass die Freigabestellung der Verriegelungsstellung entspricht und/oder die Blockierstellung der Entriegelungsstellung entspricht. Anders ausgedrückt kann der Halteabschnitt so ausgebildet sein, dass die mechanische Kopplung nur dann erfolgen kann, wenn das Einführelement verriegelt ist.

Gemäß einer Ausführungsform kann die Befestigungsanordnung so ausgebildet sein, dass zum Verbinden des Einführelements mit dem Aufnahmeelement über das Verbindungselement eine Einführbewegung, ein Abdichten und eine Verschließbewegung erfolgen. Dabei kann mit der Einführbewegung das Einführelement in das Aufnahmeelement eingeführt werden, mit dem Abdichten das Einführelement fluiddicht mit dem Aufnahmeelement verbunden werden und mit der Verschließbewegung das Einführelement im Aufnahmeelement verriegelt werden. Die Verschließbewegung kann zudem eine Relativbewegung der beweglichen Komponente relativ zum Einführelement und zum Aufnahmeelement umfassen.

Gemäß einer Ausführungsform kann die Einführbewegung zumindest teilweise in einer Richtung parallel zu einer Längsachse der Heiz- und/oder Temperaturmessvorrichtung und/oder des Einführelements erfolgen.

Gemäß einer Ausführungsform kann das Abdichten durch die Einführbewegung erfolgen.

Gemäß einer Ausführungsform kann das Abdichten entkoppelt von der Verschließbewegung erfolgen.

Gemäß einer Ausführungsform kann die Verschließbewegung zumindest teilweise in einer Richtung schräg oder quer zu einer Längsachse der Heiz- und/oder Temperaturmessvorrichtung und/oder des Einführelements erfolgen.

Gemäß einer Ausführungsform kann die Verschließbewegung eine Drehung der beweglichen Komponente relativ zum Einführelement und/oder zum Aufnahmeelement um eine Längsachse der Heiz- und/oder Temperaturmessvorrichtung und/oder des Einführelements um einen Drehwinkel zwischen 30° und 180°, bevorzugt zwischen 45° und 120°, besonders bevorzugt zwischen 80° und 100° oder zwischen 60° und 100°, umfassen. Die Verschließbewegung kann einer geraden und/oder gekrümmten Bahn folgen.

Das Abdichten kann beispielsweise allein durch die Einführbewegung entlang der Längsachse erfolgen. Die fluiddichte Verbindung des Einführelements mit dem Aufnahmeelement kann also dadurch hergestellt werden, dass das Einführelement (bis zum Anschlag in eine axiale Endstellung) in das Aufnahmeelement eingeführt wird und das Dichtelement mit einer beim Einführen aufgewandten axialen Kraft beaufschlagt wird. Somit ist zum Abdichten keine zusätzliche (axiale) Kraft erforderlich. Die Verschließbewegung kann nach dem Abdichten erfolgen. Insbesondere kann ein Spalt zwischen dem Aufnahmeelement und dem in der axialen Endstellung befindlichen Einführelement unabhängig von einer Verdrehung des Einführelements um dessen Längsachse abgedichtet sein.

Gemäß einer Ausführungsform kann die Befestigungsanordnung ferner einen Bajonettverschluss zum Verriegeln des Einführelements im Aufnahmeelement, insbesondere in der Verriegelungsstellung, umfassen. Der Bajonettverschluss kann (mindestens) ein männliches Element und (mindestens) ein zum männlichen Element komplementäres weibliches Element als Teile des Bajonettverschlusses umfassen. Das Verbindungselement, insbesondere die bewegliche Komponente, kann ein erstes der Teile des Bajonettverschlusses umfassen. Zusätzlich oder alternativ kann das Aufnahmeelement ein zweites der Teile des Bajonettverschlusses umfassen.

Zum Verriegeln des Bajonettverschlusses kann das männliche Element relativ zum weiblichen Element, in das das männliche Element eingreift, von der Entriegelungs- in die Verriegelungsstellung bewegt werden, wobei das männliche Element dem Verlauf des weiblichen Elements folgt. Eine solche Bewegung erfordert in der Regel deutlich weniger Kraftaufwand als die Drehbewegung beim Festziehen einer Schraube.

Beispielsweise kann das weibliche Element durch mindestens eine Nut und/oder das männliche Element durch mindestens einen Knopf gebildet sein. Unter "Knopf" kann allgemein eine Erhebung, insbesondere eine rundliche Erhebung, verstanden werden. Die Nut kann oder die Nuten können sich in ihrer Längsrichtung betrachtet spiralförmig in Richtung des Vorsprungs erstrecken, beispielsweise ähnlich den Furchen eines Schraubgewindes mit einer gewissen Steigung. Die Nut kann oder die Nuten können jeweils mit einer Ebene durch die Einführseite einen Winkel von 90° oder weniger, bevorzugt von 45° oder weniger, einschließen. Das weibliche Element kann beispielsweise an der Einführseite des Kanals angeordnet sein.

Gemäß einer Ausführungsform kann das Verbindungselement, insbesondere die bewegliche Komponente, als ein Überwurfelement ausgebildet sein. Das Überwurfelement kann eine auf das Einführelement aufgesetzte, relativ zum Einführelement dreh- und/oder verschiebbare Überwurfhülse, die ein Griffelement bilden kann, und das erste Teil des Bajonettverschlusses umfassen. Somit kann eine unerwünschte Relativbewegung des (verpressten) Dichtelements in Bezug auf die Heiz- und/oder Temperaturmessvorrichtung vermieden werden. Eine solche Relativbewegung kann unter Umständen dazu führen, dass das Dichtelement verrutscht oder übermäßig verschleißt, was zu Leckagen führen kann. Die Überwurfhülse kann insbesondere zwischen der Verriegelungs- und der Entriegelungsstellung und/oder am Einführelement dreh- und/oder verschiebbar gelagert sein.

Beispielsweise kann die Überwurfhülse zwei Knöpfe an einander gegenüberliegenden Seiten als männliche Elemente des Bajonettverschlusses aufweisen.

Gemäß einer Ausführungsform kann die Überwurfhülse auf den Halteabschnitt aufgesetzt sein, insbesondere derart, dass sie zumindest teilweise außerhalb des Aufnahmeelements liegt, wenn das Einführelement (beispielsweise vollständig) in das Aufnahmeelement eingeführt ist. Die Überwurfhülse kann relativ zum Halteabschnitt dreh- und/oder verschiebbar am Einführelement gelagert sein.

Gemäß einer Ausführungsform kann das Überwurfelement entlang einer Längsachse der Heiz- und/oder Temperaturmessvorrichtung und/oder des Einführelements relativ zum Halteabschnitt verschiebbar gelagert sein. In diesem Fall kann mindestens ein Halteelement an der dem Halteabschnitt zugewandten Seite des Überwurfelements ausgebildet sein, um einen Weg des Überwurfelements beim Verschieben in mindestens einer Richtung entlang der Längsachse zu begrenzen.

### Kurze Beschreibung der Zeichnungen

Im Folgenden werden beispielhafte Ausführungsformen mit Bezug auf die beigefügten Zeichnungen beschrieben. Weder die Beschreibung noch die Zeichnungen sind als Beschränkung des Umfangs der Offenbarung zu verstehen.
Fig. 1 zeigt eine Befestigungsanordnung gemäß einer Ausführungsform.
Fig. 2 zeigt eine Befestigungsanordnung gemäß einer weiteren Ausführungsform.
Fig. 3 zeigt ein Aufnahmeelement einer Befestigungsanordnung gemäß einer Ausführungsform.
Fig. 4 zeigt ein Einführelement einer Befestigungsanordnung gemäß einer Ausführungsform.
Fig. 5 zeigt ein Aufnahmeelement einer Befestigungsanordnung gemäß einer weiteren Ausführungsform.
Fig. 6 zeigt ein Einführelement einer Befestigungsanordnung gemäß einer weiteren Ausführungsform.
Fig. 7 zeigt einen Querschnitt durch das Aufnahmeelement von Fig. 5.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen

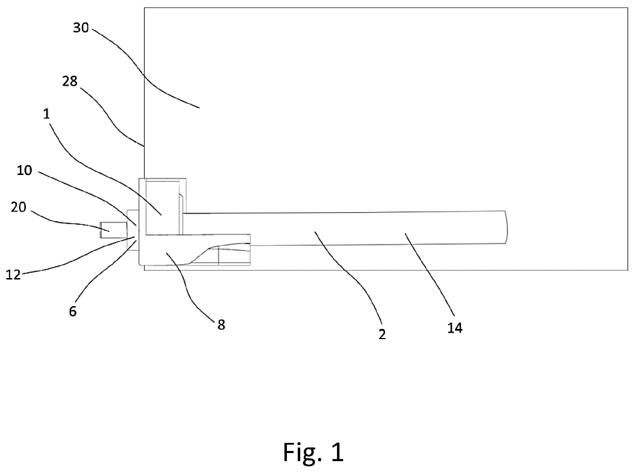

Fig. 1 zeigt eine Befestigungsanordnung 1 für eine Heizvorrichtung 2 in einem Medizingerät (hier nicht gezeigt). Dabei umfasst die Befestigungsanordnung 1 ein Einführelement 6, ein Aufnahmeelement 8 sowie ein Verbindungselement 10 zum Verbinden des Einführelements 6 und des Aufnahmeelements 8. Das Aufnahmeelement 8 ist als Teil eines Außengehäuses 28 einer Flüssigkeitskammer 30 ausgebildet, wobei die Flüssigkeitskammer 30 als Befeuchtungsvorrichtung für ein Atemtherapiegerät (hier nicht gezeigt) ausgebildet ist. Das Einführelement 6 umfasst in der gezeigten Variante eine Heizvorrichtung 2, die wiederum ein Heizelement 14 und ein elektrisches Kontaktelement 20 umfasst. Das Aufnahmeelement 8 ist so ausgebildet, dass es das Einführelement 6 zumindest teilweise aufnehmen kann, wobei hier die Heizvorrichtung 2 selbst und das elektrische Kontaktelement 20 außerhalb des Aufnahmeelements 8 liegen. Das Verbindungselement 10 weist eine relativ zum Einführelement 6 und zum Aufnahmeelement 8 bewegliche Komponente 12 auf, die das Einführelement 6 im eingeführten Zustand zumindest abschnittsweise umrahmt.

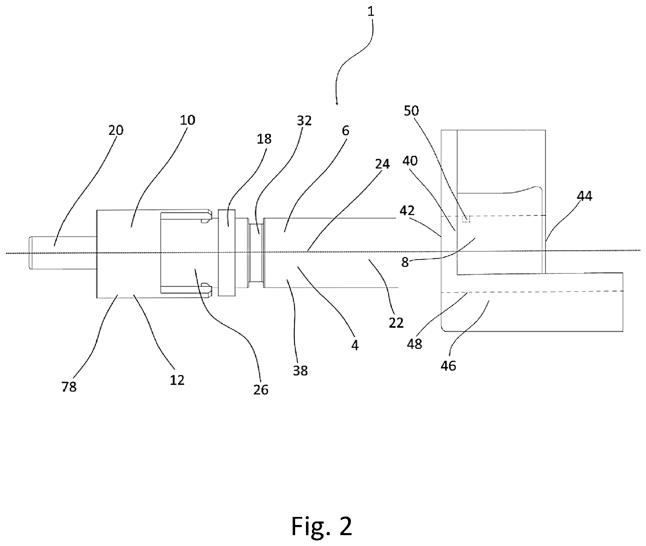

Fig. 2 zeigt eine alternative Ausführungsform der Befestigungsanordnung 1. Zur besseren Sichtbarkeit der einzelnen Komponenten sind das Einführelement 6 und das Aufnahmeelement 8 hier in einem gelösten, d. h. nicht verbundenen Zustand gezeigt. Das Aufnahmeelement 8 weist einen zylinderförmigen Kanal 40 auf, in dem das Einführelement 6 nach dem Einführen zumindest abschnittsweise liegt. Zudem umfasst das Aufnahmeelement 8 eine Einführseite 42, die dem Einführelement 6 beim Einführen zugewandt ist, und eine Begrenzungsseite 44, die dem Einführelement 6 abgewandt ist. Der Kanal 40 weist eine Innenwandung 48 und eine Ummantelung 46 auf. An der Innenwandung 48 ist in der Nähe der Begrenzungsseite 44 ein Vorsprung 50 ausgebildet, der das Einführelement 6 beim Einführen blockieren soll, insbesondere das Blockierelement 18 des Einführelements 6, sodass ein weiteres Einführen entlang der Längsachse 24 nicht mehr möglich ist. Das Einführelement 6 umfasst eine Temperaturmessvorrichtung 4, die entlang der Längsachse 24 ein elektrisches Kontaktelement 20, einen Halteabschnitt 26, ein Blockierelement 18 und einen Temperaturfühler 22 aufweist. Eine Verjüngung 32 zwischen dem Blockierelement 18 und dem Temperaturfühler 22 ist ausgebildet, um ein Dichtelement 16, insbesondere einen Dichtring 34, aufzunehmen (hier nicht gezeigt). Auf dem Halteabschnitt 26 ist das Verbindungselement 10 angebracht, das hier als eine um den Halteabschnitt 26 bewegliche Komponente 12 ausgebildet ist, die das Einführelement 6 abschnittsweise umrahmt. Die bewegliche Komponente 12 ist gleichzeitig ein Griffelement 78, an dem ein Nutzer das Einführelement 6 gefahrlos greifen kann.

Beim Einführen des Einführelements 6 in das Aufnahmeelement 8 verbindet das Verbindungselement 10 die Komponenten derart, dass eine Einführbewegung, ein Abdichten und eine Verschließbewegung erfolgt. Die Verschließbewegung umfasst eine Relativbewegung der beweglichen Komponente 12, hier eine Drehbewegung. Die Einführbewegung erfolgt hier entlang der Längsachse 24. Die Verschließbewegung erfolgt orthogonal zur Längsachse 24. Das Abdichten über das (nicht gezeigte) Dichtelement 16 erfolgt im Wesentlichen durch die Einführbewegung entlang der Längsachse 24 und im Wesentlichen entkoppelt von der Verschließbewegung.

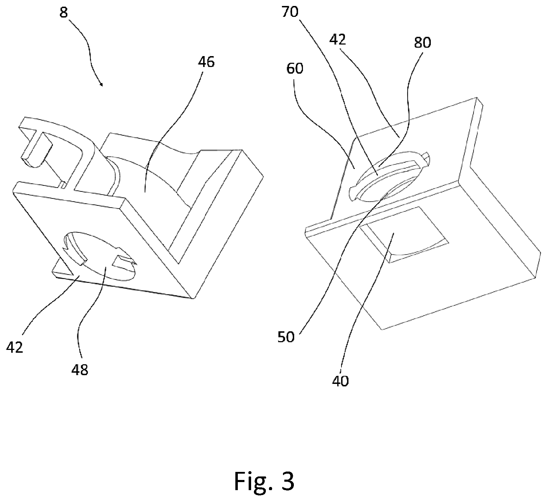

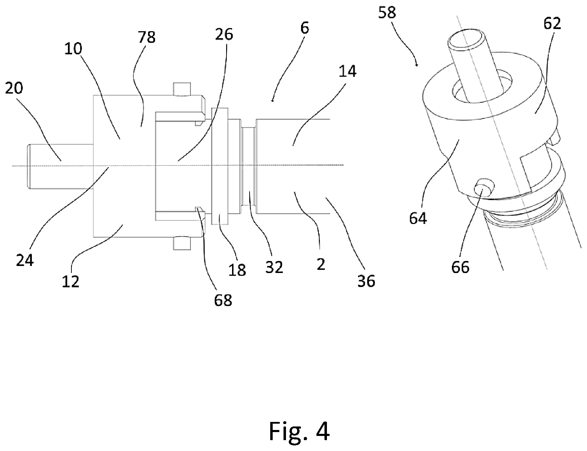

Fig. 3 zeigt eine erste Ausführungsform des Aufnahmeelements 8. Das Aufnahmeelement 8 in der hier gezeigten Variante umfasst eine Einführseite 42, die dem Einführelement 6 beim Einführen zugewandt ist, sowie eine Begrenzungsseite 44 (hier nicht gezeigt), einen Kanal 40 mit einer Innenwandung 48 und einer Ummantelung 46. An der Innenwandung 48 ist ein Vorsprung 50 ausgebildet, der das Einführelement 6 beim Einführen blockiert. Ein Teil des gezeigten Aufnahmeelements 8 bildet ein weibliches Element 60 eines Bajonettverschlusses (Gegenpart in Fig. 4) mit zwei Nuten 70, die sich spiralförmig in Richtung des Vorsprungs 50 erstrecken. Dabei schließt jede Nut 70 mit der Einführseite 42 einen Winkel 80 ein, der kleiner 45° ist.

Fig. 4 zeigt eine erste Ausführungsform des Einführelements 6. Hierbei bildet die bewegliche Komponente 12 ein männliches Element 58 des Bajonettverschlusses und ist korrespondierend zum Aufnahmeelement 8 in Fig. 3. Die bewegliche Komponente 12 ist hier als ein Überwurfelement 62 ausgebildet, das auf dem Halteabschnitt 26 bewegbar sitzt. Das Überwurfelement 62 ist als aufgesetzte, drehbare Überwurfhülse 64 ausgebildet, die gleichzeitig ein Griffelement 78 bildet, an dem ein Nutzer das Einführelement 6 sicher greifen kann. Die Überwurfhülse 64 weist zwei Knöpfe 66 auf, die von den Nuten 70 im Aufnahmeelement 8 (Fig. 3) aufgenommen werden können. Das Überwurfelement 62 ist entlang der Längsachse 24 um den Halteabschnitt 26 verschiebbar gelagert. Zwei Halteelemente 68 verhindern, dass das Überwurfelement 62 vom Halteabschnitt 26 rutscht, und begrenzen die Verschiebung entlang der Längsachse 24. Die Verschließbewegung über den Bajonettverschluss umfasst hier eine Drehbewegung um die Längsachse 24, wobei die Drehung größer als 90° ist.

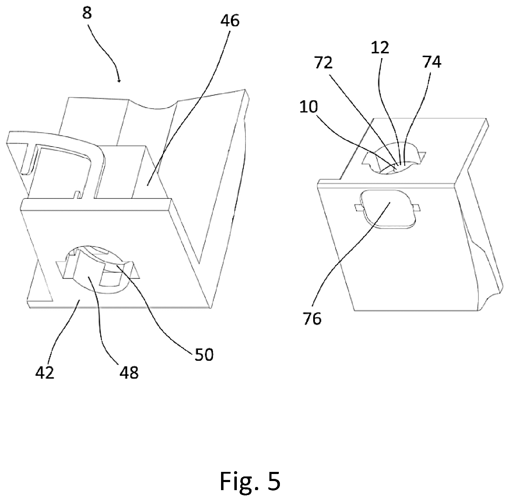

Fig. 5 zeigt eine zweite Ausführungsform des Aufnahmeelements 8. Diese unterscheidet sich von der in Fig. 3 und Fig. 4 gezeigten Variante des Bajonettverschlusses dadurch, dass das Verbindungselement 10 in Form der beweglichen Komponente 12 ein Teil des Aufnahmeelements 8 ist. Die bewegliche Komponente 12 ist hier als Einrastelement 72 zwischen der Einführseite 42 und der Begrenzungsseite 44 (hier nicht gezeigt) ausgebildet und befindet sich ausgehend von der Einführseite 42 vor dem Vorsprung 50. Das Einrastelement 72 ist als ausgehend von der Innenwandung 48 in den Kanal 40 ragender Austritt 74 ausgebildet. Beim Einführen des Einführelements 6 in das Aufnahmeelement 8 übt das Einführelement 6 Druck auf das Einrastelement 72 aus, sodass dieses in die Ummantelung 46 aufgenommen wird und nicht mehr in den Kanal 40 ragt. Alternativ oder zusätzlich kann der Druck auf das Einrastelement 72 durch Betätigen eines Druckelements 76 ausgeübt werden, das mit dem Einrastelement 72 verbunden ist und zusammen mit diesem das Verbindungselement 10, insbesondere die bewegliche Komponente 12, bildet.

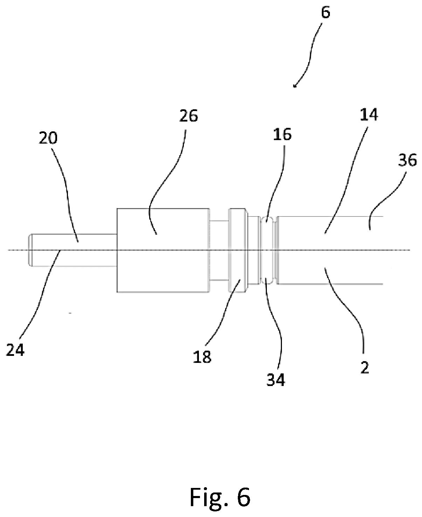

Fig. 6 zeigt eine zweite Ausführungsform des Einführelements 6. Dieses ist mit dem in Fig. 5 gezeigten Aufnahmeelement 8 kompatibel. In Richtung der Längsachse 24 betrachtet umfasst das Einführelement 6 ein elektrisches Kontaktelement 20, einen Halteabschnitt 26, ein Blockierelement 18 und ein Heizelement 14, das hier als zylinderförmiger Heizstab 36 ausgebildet ist. Zwischen dem Blockierelement 18 und dem Heizelement 14 befindet sich ein Dichtelement 16, das hier als Dichtring 34 ausgebildet ist.

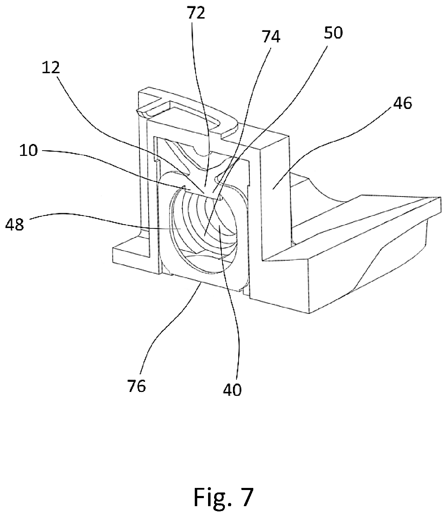

Fig. 7 zeigt einen Querschnitt durch das in Fig. 5 gezeigte Aufnahmeelement 8. Das Verbindungselement 10 ist hier als bewegliche Komponente 12 ausgebildet. Hierbei umfasst die bewegliche Komponente 12 ein Einrastelement 72 vor dem Vorsprung 50. Das Einrastelement 72 ist als ausgehend von der Innenwandung 48 in den Kanal 40 ragender Austritt 74 ausgebildet. Zusätzlich umfasst die bewegliche Komponente 12 ein Druckelement 76, das hier mit dem Einrastelement 72 verbunden ist. Durch Druck auf das Druckelement 76 fährt die bewegliche Komponente 12 derart in den Kanal 40, dass der Austritt 74 plan in der Ummantelung 46 liegt und nicht mehr in den Kanal 40 ragt.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Befestigungsanordnung
- 2: Heizvorrichtung
- 4: Temperaturmessvorrichtung
- 6: Einführelement
- 8: Aufnahmeelement
- 10: Verbindungselement
- 12: bewegliche Komponente
- 14: Heizelement
- 16: Dichtelement
- 18: Blockierelement
- 20: elektrisches Kontaktelement
- 22: Temperaturfühler
- 24: Längsachse
- 26: Halteabschnitt
- 28: Außengehäuse
- 30: Flüssigkeitskammer
- 32: Verjüngung
- 34: Dichtring
- 36: Heizstab
- 38: Tauchrohr
- 40: Kanal
- 42: Einführseite
- 44: Begrenzungsseite
- 46: Ummantelung
- 48: Innenwandung
- 50: Vorsprung
- 58: männliches Element
- 60: weibliches Element
- 62: Überwurfelement
- 64: Überwurfhülse
- 66: Knopf
- 68: Halteelement
- 70: Nut
- 72: Einrastelement
- 74: Austritt
- 76: Druckelement
- 78: Griffelement
- 80: Winkel der Nut zur Einführseite

## Patentansprüche

1. Befestigungsanordnung (1) für ein Medizingerät zur Anwendung in der Atemtherapie, wobei die Befestigungsanordnung (1) umfasst:
ein an einem Befestigungsabschnitt des Medizingeräts befestigbares Aufnahmeelement (8);
ein bis in eine Dichtstellung in das Aufnahmeelement (8) einführbares Einführelement (6), das eine Heiz- und/oder Temperaturmessvorrichtung (2, 4) zum Erwärmen und/oder Messen einer Temperatur einer Flüssigkeit und/oder eines Gases in einer Flüssigkeitskammer (30) des Medizingeräts umfasst, wobei das Einführelement (6) in der Dichtstellung fluiddicht mit dem Aufnahmeelement (8) verbunden ist;
ein Verbindungselement (10) zum Verbinden des Einführelements (6) mit dem Aufnahmeelement (8), wobei das Verbindungselement (10) eine relativ zum Aufnahmeelement (8) und/oder zum Einführelement (6) zwischen einer Verriegelungsstellung und einer Entriegelungsstellung bewegliche Komponente (12) umfasst, wobei die bewegliche Komponente (12) ausgebildet ist, um das bis in die Dichtstellung in das Aufnahmeelement (8) eingeführte Einführelement (6) in der Verriegelungsstellung zu verriegeln, sodass ein Entnehmen des Einführelements (6) aus dem Aufnahmeelement (8) nicht möglich ist, und in der Entriegelungsstellung freizugeben, sodass das Entnehmen möglich ist, wobei die bewegliche Komponente (12) relativ zum Aufnahmeelement (8) und/oder zum Einführelement (6) zwischen der Verriegelungsstellung und der Entriegelungsstellung um einen Drehwinkel von höchstens 360°, höchstens 180° oder höchstens 90° um eine Drehachse (24) drehbar gelagert ist.

2. Befestigungsanordnung (1) nach Anspruch 1,
wobei die bewegliche Komponente (12) ferner relativ zum Aufnahmeelement (8) und/oder zum Einführelement (6) zwischen der Verriegelungsstellung und der Entriegelungsstellung verschiebbar gelagert ist.

3. Befestigungsanordnung (1) nach einem der vorhergehenden Ansprüche,
wobei - wenn das Einführelement (6) bis in die Dichtstellung in das Aufnahmeelement (8) eingeführt ist - ein Spalt zwischen dem Einführelement (6) und dem Aufnahmeelement (8) so abgedichtet ist, dass keine Flüssigkeit und/oder kein Gas aus der Flüssigkeitskammer (30) über den Spalt nach außen entweichen kann, wobei die Befestigungsanordnung (1) ferner ein Dichtelement (16) zum Abdichten des Spalts umfasst und so ausgebildet ist, dass das Dichtelement (16) beim Einführen des Einführelements (6) bis in die Dichtstellung einerseits durch das Einführelement (6) und/oder die bewegliche Komponente (12) und andererseits durch das Aufnahmeelement (8) zusammengedrückt wird, um das Einführelement (6) fluiddicht mit dem Aufnahmeelement (8) zu verbinden.

4. Befestigungsanordnung (1) nach einem der vorhergehenden Ansprüche,
wobei die Befestigungsanordnung (1) so ausgebildet ist, dass das Einführen des Einführelements (6) bis in die Dichtstellung ohne Drehen des Einführelements (6) relativ zum Aufnahmeelement (8) um mehr als 360°, bevorzugt mehr als 180°, besonders bevorzugt mehr als 90°, um die Drehachse (24) möglich ist, oder
wobei die Befestigungsanordnung (1) so ausgebildet ist, dass das Einführelement (6) beim Einführen ausschließlich in einer Richtung parallel zu dessen Längsachse bewegt wird.

5. Befestigungsanordnung (1) nach einem der vorhergehenden Ansprüche,
wobei die bewegliche Komponente (12) mindestens ein Einrastelement (72) zum Verrasten des Einführelements (6) mit dem Aufnahmeelement (8) umfasst, wobei das mindestens eine Einrastelement (72) ausgebildet ist, um das Einführelement (6) mit dem Aufnahmeelement (8) beim Bewegen der beweglichen Komponente (12) in die Verriegelungsstellung zu verrasten.

6. Befestigungsanordnung (1) nach einem der vorhergehenden Ansprüche,
wobei die bewegliche Komponente (12) ferner einen Halteabschnitt (26) zum Halten der beweglichen Komponente (12) mit einer Hand umfasst, wobei der Halteabschnitt (26) so ausgebildet ist, dass er zumindest teilweise außerhalb des Aufnahmeelements (8) liegt, wenn das Einführelement (6) in das Aufnahmeelement (8) eingeführt ist.

7. Befestigungsanordnung (1) nach Anspruch 6,
wobei der Halteabschnitt (26) einen ringförmigen Grundkörper zum Aufschieben auf das Einführelement (6) oder auf die Heiz- und/oder Temperaturmessvorrichtung (2, 4) und einen in radialer Richtung vom Grundkörper abstehenden Haltearm zum Bewegen des Grundkörpers relativ zum Einführelement (6) und/oder zum Aufnahmeelement (8) umfasst.

8. Befestigungsanordnung (1) nach Anspruch 7,
wobei der Haltearm an seinem freien Ende ein flächiges Griffelement zum Greifen des Haltearms mit einem Daumen und einem Zeigefinger aufweist oder fahnenartig ausgebildet ist.

9. Befestigungsanordnung (1) nach einem der vorhergehenden Ansprüche,
wobei das Aufnahmeelement (8) als Teil eines Außengehäuses der Flüssigkeitskammer (30) ausgebildet ist.

10. Befestigungsanordnung (1) nach einem der vorhergehenden Ansprüche, ferner umfassend:
einen Bajonettverschluss zum Verriegeln des Einführelements (6) im Aufnahmeelement (8) in der Verriegelungsstellung, wobei der Bajonettverschluss ein männliches Element (58) und ein zum männlichen Element (58) komplementäres weibliches Element (60) als Teile des Bajonettverschlusses umfasst, wobei die bewegliche Komponente (12) ein erstes der Teile des Bajonettverschlusses umfasst und das Aufnahmeelement (8) ein zweites der Teile des Bajonettverschlusses umfasst, wobei zum Verriegeln des Bajonettverschlusses das weibliche Element (60) relativ zum in das weibliche Element (60) eingreifenden männlichen Element (58) von der Entriegelungsstellung in die Verriegelungsstellung bewegbar ist, wobei das männliche Element (58) dem Verlauf des weiblichen Elements (60) folgt.

11. Befestigungsanordnung (1) nach Anspruch 10,
wobei die bewegliche Komponente (12) als ein Überwurfelement (62) ausgebildet ist, wobei das Überwurfelement (62) eine auf das Einführelement (6) aufgesetzte, relativ zum Einführelement (6) zwischen der Verriegelungsstellung und der Entriegelungsstellung dreh- und/oder verschiebbare Überwurfhülse (64) und das erste Teil des Bajonettverschlusses umfasst.

12. Befestigungsanordnung (1) nach Anspruch 11,
wobei die Überwurfhülse (64) am Einführelement (6) dreh- und/oder verschiebbar gelagert ist.

13. Medizingerät zur Anwendung in der Atemtherapie, umfassend:
eine Flüssigkeitskammer (30);
einen Befestigungsabschnitt;
eine Befestigungsanordnung (1) nach einem der vorhergehenden Ansprüche, wobei das Aufnahmeelement (8) der Befestigungsanordnung (1) am Befestigungsabschnitt befestigt ist.

14. Medizingerät nach Anspruch 13,
wobei das Aufnahmeelement (8) gegenüber einer Öffnung in einem Gehäuse (28) der Flüssigkeitskammer (30) derart ausgerichtet ist, dass die Heiz- und/oder Temperaturmessvorrichtung (2, 4) durch die Öffnung hindurch ins Innere der Flüssigkeitskammer (30) ragt, wenn das Einführelement (6) vollständig in das Aufnahmeelement (8) eingeführt ist.

15. Medizingerät nach Anspruch 13 oder 14,
wobei die Befestigungsanordnung (1) eine Befestigungsanordnung (1) nach einem der Ansprüche 6 bis 8 ist, wobei der Halteabschnitt relativ zum Aufnahmeelement (8) und/oder zum Einführelement (6) zwischen einer Blockierstellung und einer Freigabestellung bewegbar ist und ausgebildet ist, um eine mechanische Kopplung des Medizingeräts mit einem weiteren Gerät, vorzugsweise einem Atemluftbefeuchter, in der Blockierstellung zu blockieren und in der Freigabestellung zu ermöglichen, wobei die Freigabestellung der Verriegelungsstellung der beweglichen Komponente (12) entspricht.
